# EUROPÄISCHE PATENTSCHRIFT

(11) **EP 0 547 200 B1**
(45) Veröffentlichungstag und Bekanntmachung des Hinweises auf die Patenterteilung: **29.04.1998**
(21) Anmeldenummer: 92914024.2
(22) Anmeldetag: 06.07.1992
(51) Int. Cl.: C12N 15/13, C12P 21/08, C12Q 1/68, C07K 16/28, G01N 33/53

(54) **REKOMBINANTE ANTIKÖRPER AN DER OBERFLÄCHE VON E.COLI**
RECOMBINANT ANTIBODIES AT THE SURFACE OF E. COLI
ANTICORPS RECOMBINANTS A LA SURFACE D'E. COLI

(30) Priorität: 08.07.1991 DE 4122598
(43) Veröffentlichungstag der Anmeldung: 23.06.1993
(73) Patentinhaber: Deutsches Krebsforschungszentrum Stiftung des öffentlichen Rechts, 69120 Heidelberg (DE)
(72) Erfinder: FUCHS, Patrick, D-6900 Heidelberg (DE); LITTLE, Melvyn, D-6903 Neckargemünd-Dilsberg (DE); BREITLING, Frank, D-6900 Heidelberg (DE); DÜBEL, Stefan, D-6900 Heidelberg (DE)
(74) Vertreter: Schüssler, Andrea, Dr.
(86) Internationale Anmeldenummer: EP9201523
(87) Internationale Veröffentlichungsnummer: WO9301287

(56) Entgegenhaltungen:
- PROTEINS: STRUCTURE, FUNCTION AND GENETICS Bd. 8, Nr. 4, 1990, WILEY-PRESS,NY, US; Seiten 309 - 314 S. BASS ET AL. 'Hormone phage: An enrichment method for variant proteins with altered binding properties'
- EUR. J. BIOCHEM. Bd. 163, Nr. 1, Februar 1987, SPRINGER VERLAG, BERLIN, BRD; Seiten 73 - 77 R.CHEN AND U.HENNING 'Nucleotide sequence of the gene for the peptidoglycan-associated lipoprotein of Escherichia coli K12' in der Anmeldung erw hnt
- NATURE Bd. 349, 24. Januar 1991, MACMILLAN JOURNALS LTD., LONDON, UK; Seiten 293 -299 G. WINTER AND C. MILSTEIN man-made antibodies
- BIO/TECHNOLOGY Bd. 9, Nr. 12, Dezember 1991, NATURE AMERICA, INC., NEW YORK, US Seiten 1369 - 1372 P. FUCHS ET AL. 'Targeting recombinant antibodies to the surface of Escherichia coli: fusion to a peptidoglycan associated lipoprotein'

## Beschreibung

Die vorliegende Erfindung betrifft Vektoren, die Einzelketten-Antikörper exprimieren, die mit der Oberfläche von Antikörper-produzierenden Zellen gekoppelt sind. Ferner betrifft die Erfindung die Verwendung dieser Vektoren zur raschen Isolierung einzelner spezifischer Antikörper-produzierender Zellen. Schließlich betrifft die Erfindung Verfahren zur Isolierung von spezifischen Antikörper-produzierenden Zellen.

Frühere Verfahren zum Screenen von rekombinanten Antikörpern haben ELISA-Tests von bakteriellen Überständen (Nature (1989), 341, 544-546) oder radioaktiv markierte Immunogene zum Screenen von auf Nitrocellulose übertragenen Plaques von mit Phagen-Expressionsvektoren infizierten, bakteriellen Kolonien vorgesehen (Science (1989), 246, 1275-1281; Proc. Natl. Acad. Sci. USA (1990), 87, 6450-6454; Proc. Natl. Acad. Sci. USA (1990), 87, 8095-8099). Zur Selektion von spezifischen Antikörpern aus Genbanken willkürlich kombinierter, leichter und schwerer Ketten, die kein Übergewicht von Antikörpern für ein bestimmtes Antigen haben, würde das Screenen von Millionen von Antikörperklonen jedoch stark erleichtert werden, wenn Antikörper gezielt auf die Oberfläche von Bakterien oder Viren gebracht werden. Immobilisierte Antigene könnten dann zur Selektion von spezifischen Antikörpern verwendet werden.

Ein virales System für die Oberflächenpräsentation von Antikörpern wurde kürzlich in Nature (1990), 348, 552-554 beschrieben. Variable Domänen von Einzelketten (Vorläufer des Antikörperkonstruktes der Anmelderin) wurden mit dem Anheftungsprotein (Protein p III) von Phagenpartikeln fusioniert. Phagen, die das Fusionsprotein trugen, konnten an Antigen-Säulen angereichert werden.

Es zeigte sich jedoch, daß diese Fusionsphagen hauptsächlich für relativ kleine Inserts geeignet sind, da wahrscheinlich die größeren Inserts einen negativen Effekt auf die Infektivität von p III haben. Genbanken von "Phagen-Antikörpern" beinhalten daher die Gefahr, daß Deletionsmutanten rasch dominant werden. Ferner scheint eine relativ große Anzahl von Phagenpartikeln unspezifisch an Säulen immobilisierten Antigens zu binden.

Der vorliegenden Erfindung liegt daher die Aufgabe zugrunde, ein effizienteres Mittel zum Screenen von Antikörper-Genbanken in Bakterien bereitzustellen.

Erfindungsgemäß wird dies durch einen Vektor erreicht, der variable Domänen von Einzelketten-Antikörpern, gekoppelt an das mit Peptidoglycan assoziierte Lipoprotein von E.coli (PAL, J. Biochem. (1979), 86, 991-1000; Eur. J. Biochem. (1987), 163, 73-77), exprimiert.

PAL ist eine Zellhüllen-Komponente von E.coli, die besonders resistent gegen SDS-Aufschluß ist (J. Biochem. (1979), 86, 991-1000). Ihre Peptidoglycan assoziierte Protein-Komponente hat ein Molekulargewicht von 16600 (Eur. J. Biochem. (1987), 163, 73-77) und ist am Amino-terminalen Cystein durch einen in der äußeren Membran integrierten Lipidteil modifiziert. Die Bindung von Antikörpern an den Amino-Terminus von PAL ist daher eine Möglichkeit, sie zur Antigen-Bindung an der Zelloberfläche zu präsentieren.

Das Antikörper-PAL-Fusionsprotein wurde auf der Zelloberfläche durch einen monoklonalen Antikörper identifiziert, der gegen ein Epitop in der Linker-Sequenz zwischen den schweren und leichten Ketten gerichtet ist. Es konnte Antigen binden und war fest an die Mureinschicht der Zellhülle gebunden. Immunfluoreszenzstudien an nicht-fixierten Zellen zeigten, daß funktionelle Antikörper-Domänen gut an der Zelloberfläche zugänglich waren.

Ein großer Reiz des erfindungsgemäßen bakteriellen Systems liegt darin, daß ein Menge von Antikörpermolekülen zur Bindung verfügbar sind, im Gegensatz zu den fünf Antikörper-Fusionsmolekülen, die pro Partikel im Phagensystem gebunden werden. Die Verwendung von Immunfluoreszenz markierten Antigenen sollte deshalb ein rasches Mittel zur Isolierung von spezifische Antikörper-produzierenden Zellen darstellen. Beispielsweise könnte Screenen mit einem Fluoreszenz-Zellsorter (FACS) theoretisch eine Anreicherung von mindestens 10⁶ in einem Schritt bedeuten, da mehrere Millionen Bakterien gleichzeitig abgetastet und einzelne Bakterien selektiert werden können. Andererseits könnten spezifische Antikörper-produzierende Bakterien durch Bindung an immobilisierte Antigene selektiert werden. Weitere Vorteile umfassen die Transformations-Effizienz, die Leichtigkeit der Vermehrung und das Fehlen eines Selektionsdrucks auf die Entfernung von Antikörper-DNA während der Genbank-Amplifikation.

Eine bevorzugte Verwendung der vorliegenden Erfindung liegt darin, Antikörper gegen Tumor-assoziierte Antigene rasch durch unterschiedliche Screening-Tests zu isolieren. Beispielsweise sollten nach einer ersten Abreicherung einer Antikörper-Genbank auf Zelloberflächen-Antigenen normalen Gewebes die Zellen neoplatischen Gewebes nur jene Antikörper binden, die für Unterschiede spezifisch sind. Dieses Verfahren könnte ebenfalls zum Aufdecken antigener Unterschiede zwischen nah verwandten Zellen oder Organellen herangezogen werden.
Das erfindungsgemäße PAL-Vektorsystem könnte sich ebenfalls als nützlich erweisen, andere Proteine und Peptide an der Oberfläche von E.coli zu präsentieren. Dies könnte dann die Produktion von Lebendvakzinen erleichtern. Beispielsweise wurden Epitope, die in Zelloberflächen-Loops des in der äußeren Membran grammnegativer Bakterien vorliegenden LamB-Proteins inseriert waren, zur Herstellung von Antikörpern verwendet. Hierfür könnte sich das offensichtliche Fehlen eines Effekts auf das bakterielle Wachstum durch das überexprimierte PAL-Fusionsprotein als besonders vorteilhaft erweisen.

Die nachfolgenden Beispiele erläutern die Erfindung.

### Beispiel 1

Zur Herstellung eines Antikörper-PAL-Fusionsproteins wurde eine DNA verwendet, die für die variablen Domänen der schweren und leichten Kette eines humanisierten Antikörpers gegen Hühnerlysozym (beschrieben in Nature (1988), 332, 323-327), woran achtzehn das Epitop des Tubulin monoklonalen Antikörpers YOL1/34 (J. Mol. Biol. (1986), 189, 367-370) enthaltende Aminosäuren gebunden waren, kodierte. Neben der Bereitstellung als Identifikationsmittel wurde die Linker-Sequenz zugegeben, um die Dimerisierung der zwei Ketten zu erleichtern und ihre Dissoziation zu verhindern.

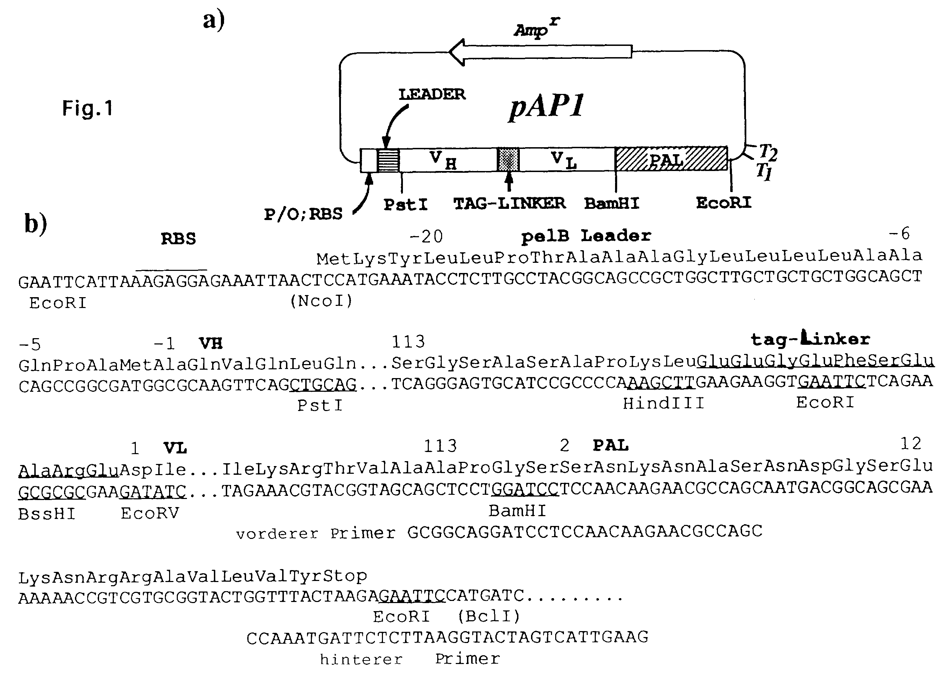

Eine flexible Verbindung mit PAL wurde erreicht, indem die leichte Kette verlängert wurde, wodurch sie die ersten sechs Aminosäuren der konstanten Domäne umfaßte. Zur Erleichterung des Transports durch die cytoplasmatische Membran wurde eine für die Leader-Sequenz des Enzyms Pectat Lyase kodierende DNA mit dem 5'-Ende der DNA für die schwere Kette (Lei et al., 1987; Better et al., 1988) ligiert und PAL-DNA, die im Plasmid pRC2 (Chen and Henning, 1987) amplifiziert worden war, wurde dann mit der Antikörper-DNA (Fig. 1) verbunden.

Ein kleiner Unterschied zwischen dem natürlichen und in vitro synthetisierten PAL lag im Austausch des amino-terminalen, normalerweise den Lipidteil tragende Cystein durch Glycin.

### Beispiel 2

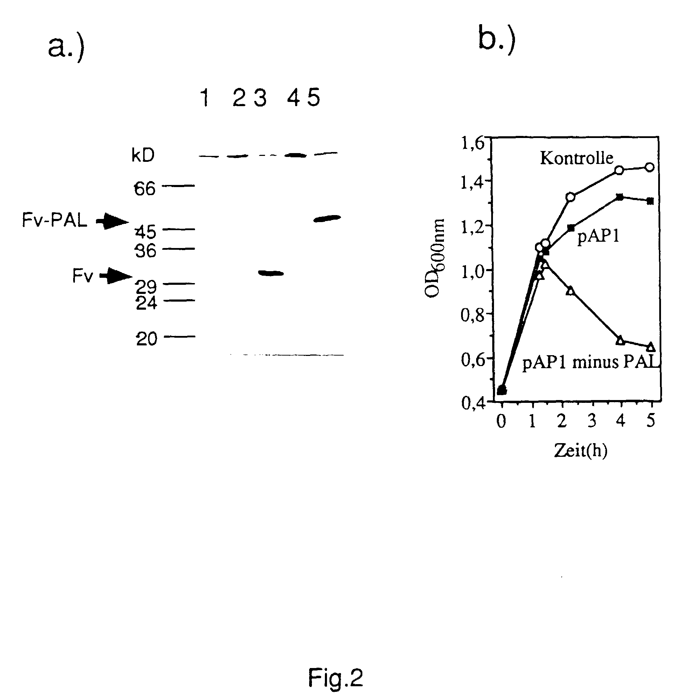

Zur Prüfung, ob das Fusionsprotein in voller Länge exprimiert werden konnte, wurde 1 mM IPTG einer sich in Log-Phase befindlichen, mit pAP1 transformierten E.coli-Kultur zugegeben. In Western Blots von SDS-Polyacrylamidgelen wurde das Antikörper-PAL-Fusionsprotein durch einen monoklonalen Antikörper identifiziert, der gegen das Marker-Peptid in der Linker-Sequenz zwischen den schweren und leichten Ketten (Fig. 2a) gerichtet war. Sein offensichtliches Molekulargewicht von etwa 48 kd war etwas höher als das vorhergesagte von Mr 45000, was wahrscheinlich an der abweichenden elektrophoretischen Beweglichkeit von PAL lag. Das Vorliegen einer schwächeren Bande niedrigeren Molekulargewichts deutete auf einen geringen Grad an Proteolyse hin. Eine cytoplasmatische E.coli-Komponente höheren Molekulargewichts, die mit YOL1/34 kreuzreagierte, wurde in allen zellen gefunden. Vergleiche der Wachstumsraten von E.coli, die mit pAPl und pAPl ohne Antikörper-PAL-DNA transformiert waren, zeigten nach Zugabe von IPTG kleine Unterschiede innerhalb eines Zeitraums von 5 h. Im Gegensatz dazu begann eine identische, mit pAPl ohne PAL-DNA transformierte E.coli Kultur, die nur einen Einzelketten-Antikörper exprimierte, nach 2 h zu lysieren (Fig. 2b).

### Beispiel 3

Zur Prüfung der Bindung des Fusionsproteins an die Zellwand wurden Bakterien durch Schütteln mit Glasperlen in einem Zellzerkleinerer aufgebrochen und mit 1% SDS bei 10°C extrahiert. Das restliche Material wurde 1 h bei 30°C mit 1% SDS und dann 1 h bei 50°C mit 1% SDS inkubiert. Eine Western Blot-Analyse und angefärbte Polyacrylamidgele zeigten, daß zwar etwas von dem Fusionsprotein bei 30°C entfernt wurde, daß aber der Hauptanteil höhere Temperaturen benötigte, um freigesetzt zu werden. Diese Bindungseigenschaften waren sehr ähnlich jenen, die für PAL alleine beschrieben wurden (J. Biochem. (1979), 86, 991-1000; J. Biochem. (1979), 86, 979-989) und ermöglichten das Herstellen hoch angereicherter Proben in wenigen Verfahrensschritten. Natives PAL-Protein wandert mit einem offensichtlichen Molekulargewicht von etwa 20 kd und entspricht wahrscheinlich einem Protein diesen Molekulargewichts, das mit dem Fusionsprotein co-gereinigt wurde. Densitometrischer Vergleich der Mengen des Fusionsproteins mit denen des vermeintlichen PAL-Proteins wiesen daraufhin, daß etwa fünfmal mehr Fusionsprotein als natives PAL vorlagen.

Überraschenderweise wurde ein kleiner Teil des Fusionsproteins in das Medium freigesetzt, sogar schon zu Beginn der logarythmischen Phase, wo noch keine Zeichen von Zelllyse entdeckt werden konnten. Dies könnte möglicherweise an der Sättigung von PAL-Bindungsstellen und an einem erleichterten Transport von Antikörper-Domänen durch die äußere Membran oder an einem Loch in dieser liegen. Zur Bestimmung, ob das Fusionsprotein Antigen binden konnte, wurde das Medium über eine Säule von an Sepharose gekoppeltem Lysozym gegeben. Gelelektrophorese und Western Blot des ungebundenen Materials und der nach intensivem Waschen und Eluieren mit 0,05 M Diethylamin erhaltenen Fraktionen zeigte, daß das Fusionsprotein tatsächlich spezifisch an der Lysozymsäule zurückgehalten wurde.

### Beispiel 4

Die Zugänglichkeit des gebundenen Antikörpers für extrazelluläre Proteine an der Oberfläche nicht-fixierter Zellen wurde getestet, indem E.coli-Zellen mit dem monoklonalen Antikörper YOL1/34 und dann mit einem fluoreszenzmarkierten Anti-Rattenserum inkubiert wurden. E.coli-Zellen, die das Antikörper-PAL Fusionsprotein exprimierten, zeigten eine starke Fluoreszenz, die am Rand und an den Verbindungen innerhalb kurzer Ketten besonders stark war. Im Gegensatz dazu zeigten E.coli-Zellen, die das Fusionsprotein nicht exprimierten, keine Fluoreszenz. Experimente mit fixierten Zellen ergaben die gleichen Resultate. Die gute Zugänglichkeit an der Zelloberfläche mag nicht nur das gezielte Anbringen von PAL auf der äußeren Membran und der Neigung von Antikörper-Domänen, die Membran zu durchqueren, bedingt sein, sondern auch auf dem Effekt von PAL auf die äußere Membranstruktur beruhen. Messungen von β-Lactamase, einem löslichen periplasmatischen Protein, ergaben, daß hiervon im Medium von Zellen, die das Fusionsprotein exprimierten, weitaus mehr vorlag, als im Medium von Zellen, die das Fusionsprotein nicht exprimierten.

Inkubationen von nicht-fixierten Zellen mit dem biotinylierten Antigen, nämlich Hühner-Lysozym, was etwas schädlich für die zelluläre Integrität ist, demonstrierten die Fähigkeit des Fusionsproteins, Antigen in situ zu binden. Nach intensivem Waschen und Inkubation mit fluoreszierendem Avidin, zeigten E.coli-pAPl eine bezeichnende Fluoreszenz. Im Gegensatz dazu zeigten E.coli, die mit pAPl ohne Antikörper-PAL-DNA transformiert waren, keine Fluoreszenz.

Hinsichtlich der Zugänglichkeit funktioneller Antikörper-Domänen an der Zelloberfläche wurden E.coli-Zellen, die mit pAPl ohne Antikörper-PAL-DNA bzw. mit pAPl transformiert waren, mit YOL1/34 und einem zweiten Fluorescein-markierten Antikörper oder mit biotinyliertem Lysozym und einem Fluorescein-markierten Avidin inkubiert.

### Durchführung:

Zellen wurden in PBS gewaschen und zum Absetzen auf Poly-L-Lysin-beschichtete Objektträger 20 min bei 4°C inkubiert. Sie wurden dann 1 h bei 37°C mit dem monoklonalen Antikörper YOL1/34, verdünnt 1:100 in PBS, inkubiert. Nach Waschen in PBS wurden sie 1 h bei 37°C mit Anti-Ratten-IgG-FITC, verdünnt 1:100, inkubiert und erneut in PBS gewaschen. Zur Antigenbindung wurde Hühnerlysozym biotinyliert, 1:20 in PBS verdünnt und mit den Zellen 1 h bei 37°C inkubiert. Nach intensivem Waschen mit PBS wurden die zellen 1 h bei 37°C mit FITC-Avidin, verdünnt 1:1000, inkubiert und erneut in PBS gewaschen.

Es sollte auch möglich sein, andere Proteine und Peptide an die Zellwand zu binden, mit der Maßgabe, daß sie sekretiert werden können, wenn sie mit einer bakteriellen Leader-Sequenz fusioniert werden. Dies könnte dann die Produktion von Lebendvakzinen erleichtern. Hierfür könnte das offensichtliche Fehlen eines Effekts auf das bakterielle Wachstum durch das überexprimierte PAL-Fusionsprotein besonders vorteilhaft sein.

### Beschreibung der Figuren

### Fig. 1: Antikörper-PAL Expressionsplasmid pAPl

P/O: Promoter/Operator; RBS: Ribosomenbindungsstelle; Leader: Signalsequenz der Pectatlyase; Tag-Linker: 18 Aminosäuren, die das Epitop für den monoklonalen Tubulin-Antikörper YOL1/34 enthalten; PAL: Peptidoglycan-assoziiertes Protein.

### Konstruktion:

DNAs, die für die variablen Domänen der schweren und leichten Kette eines humanisierten Hühner-Lysozym-Antikörpers, der von dem monoklonalen Antikörper D 1.3 (Amit et al., Science (1986), 233, 747-754) abstammte, codierten, wurden durch eine für 18 Aminosäuren (GSASAPKLEEGEFSEARE) codierende DNA, die das Epitop für YOL1/34 enthielt, verbunden. Die DNA für die leichte Kette wurde verlängert, um Nucleotide zu umfassen, die für die ersten 6 Aminosäuren der konstante Domäne codieren. Diese wurde dann mit dem 3'-Ende der für die Signalsequenz des Enzyms Pectatlyase codierenden DNA ligiert, die in die Ncol-Stelle des geringfügig modifizierten Expressionsplasmids pKK233-2 (Clontech, Palo Alto, Ca, USA) inseriert worden war. PAL-DNA, die unter Verwendung von Primern wie in Fig. 1 gezeigt, in dem Plasmid pRC2 amplifiziert worden war, wurde dann mit der Antikörper-DNA verbunden. Die Sequenz-des inserierten PALs war identisch mit dem nativen PAL, außer, daß das aminoterminale Cystein durch Glycin ersetzt worden war.

### Fig. 2: Expression und Bindungseigenschaften des Antikörper-PAL Fusionsproteins

(a) Western Blots von induziertem Fusionsprotein nach Zugabe von 1mM IPTG. 1, pAPl ohne Antikörper-PAl DNA (+IPTG); 2, pAPl ohne PAL DNA (-IPTG); 3, pAPl ohne PAL DNA (+ IPTG); 4, pAPl (-IPTG); 5, pAPl (+IPTG).
(b) Wachstumskurve von E.coli, transformiert mit pAPl, pAPl ohne PAL DNA und pAPl ohne Antikörper-PAL DNA (Kontrolle) und nach Induktion mit 1mM IPTG.

### Induktion und Extraktion von Antikörper-PAL:

Der pDMI-tragende E.coli-Stamm BMH71/18, der den lac-Repressor exprimiert, wurde mit pAPl und pAPl-Vorläufern transformiert. Zellen wurden in 125 ml LB-Medium bis zu einer OD von 0,45 bei 600 nm kultiviert, dann mit lmM IPTG induziert und nach 1,5 h geerntet. Zur SDS-Extraktion wurden die Zellen in 3 ml H₂O suspendiert und aufgebrochen, indem sie in einem Zellzerstörer 5 min mit 5 ml Glasperlen (Durchmesser 0,13 mm) und 0,5 mg DNase 1 geschüttelt wurden. Nach Einstellen auf 1% SDS in 10mM Tris, pH 7,5, wurde das Zelllysat bei 42000 g 45 min bei 10°C sedimentiert. Das Pellet wurde in 1% SDS/10mM Tris/10% Glycerin, pH 7,8 1 h bei 30°C resuspendiert und wie vorher sedimentiert. Dieser Schritt wurde bei 50°C wiederholt. Fusionsproteine wurden auf Western Blots mit dem monoklonalen Antikörper YOL1/34 nachgewiesen. Vor der Antikörper-Anfärbung wurden die Blots für Proteine mit Ponceau S. angefärbt. Polyacrylamidgele wurden mit Coomassie blau angefärbt. Zur Affinitäts-Chromatographie wurde Hühner-Lysozym an Cyanogenbromid-aktivierte Sepharose gemäß den Vorschriften des Herstellers gekoppelt. Die Lysozym-Sepharose wurde 20 min bei Raumtemperatur mit 10 ml Medium inkubiert und in Säulen gegossen, die nachfolgend zweimal mit 10 Badvolumina PBS gewaschen wurden, bevor sie mit 0,05 M Diethylamin eluiert wurden.

## Patentansprüche

1. Vektor, dadurch gekennzeichnet, daß er eine Einzelkette von Antikörper-variablen Domänen gekoppelt an das mit Peptidoglycan assoziierte Lipoprotein (PAL) von E.coli exprimiert.

2. Vektor nach Anspruch 1, dadurch gekennzeichnet, daß er das Plasmid pAPl von Figur 1 ist.

3. Verwendung des Vektors nach Anspruch 1 oder 2 zur raschen Isolierung einzelner spezifischer Antikörper-produzierender Zellen.

4. Verfahren zur Isolierung von spezifischen Antikörper-produzierenden Zellen, bei dem man
(a) Bakterien mit einem Vektor nach Anspruch 1 oder 2 transformiert,
(b) Bakterien, die spezifische Antikörper an der Zelloberfläche exprimieren, durch fluoreszenzvermitteltes Zellsorten oder durch Bindung an immobilisierte Antigene selektiert.

5. Verwendung des Expressionsvektors nach Anspruch 1 oder 2 zur Präsentation anderer Proteine oder Peptide auf der Oberfläche von Bakterien, indem die Antikörper-DNA durch DNA des gewünschten Polypeptids ersetzt wird.

## Claims

1. A vector, characterized in that it expresses a single chain of antibody variable domains coupled to the peptidoglycan associated lipoprotein (PAL) of *E. coli.*

2. The vector according to claim 1, characterized in that it is the plasmid pAP1 described in fig. 1.

3. Use of the vector according to claim 1 or 2 for the rapid isolation of individual specific cells producing antibodies.

4. A method for the isolation of specific cells producing antibodies, which comprises
(a) transforming bacteria with a vector according to claim 1 or 2,
(b) selecting bacteria expressing specific antibodies on the cell surface by fluorescence assisted cell sorting or by binding to immobilized antigens.

5. Use of the expression vector according to claim 1 or 2 for the presentation of other proteins or peptides on the surface of bacteria by replacing the antibody DNA by DNA of the desired polypeptide.

## Revendications

1. Vecteur, caractérisé en ce qu'il exprime une chaîne particulière d'anticorps de domaines variables couplée à la lipoprotéine (PAL) associée à la peptidoglycane de E. coli.

2. Vecteur selon la revendication 1, caractérisé en ce qu'il est le plasmide pAP1 de la figure 1.

3. Application d'un vecteur selon la revendication 1 ou 2 pour l'isolation rapide de cellules particulières productrices d'anticorps spécifiques.

4. Procédé d'isolation de cellules produisant des anticorps spécifiques, par lequel
(a) on transforme des bactéries avec un vecteur selon la revendication 1 ou 2,
(b) on sélectionne des bactéries qui expriment des anticorps spécifiques à la surface de la cellule par une espèce de cellule produite par fluorescence ou par liaison des antigènes immobilisés.

5. Application du vecteur d'expression selon la revendication 1 ou 2 pour la présentation d'autres protéines ou peptides à la surface de bactéries, où l'ADN de l'anticorps est remplacé par l'ADN du polypeptide désiré.
